(19) Europäisches Patentamt / European Patent Office / Office européen des brevets

(11) **EP 4 509 168 A1**

(12) **EUROPEAN PATENT APPLICATION**
published in accordance with Art. 153(4) EPC

(43) Date of publication:
**19.02.2025 Bulletin 2025/08**

(21) Application number: **23803296.5**

(22) Date of filing: **06.04.2023**

(51) International Patent Classification (IPC):
***A61N 2/04*** (2006.01)

(52) Cooperative Patent Classification (CPC):
**A61N 2/02**

(86) International application number:
**PCT/JP2023/014178**

(87) International publication number:
**WO 2023/218813 (16.11.2023 Gazette 2023/46)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **11.05.2022 JP 2022077897**

(71) Applicant: **Nipro Corporation**
**Osaka 566-8510 (JP)**

(72) Inventors:
• **AOYAMA, Masato**
  **Settsu-shi, Osaka 566-8510 (JP)**
• **FUKUHARA, Kayoko**
  **Settsu-shi, Osaka 566-8510 (JP)**
• **HASHIGUCHI, Seishin**
  **Settsu-shi, Osaka 566-8510 (JP)**
• **OHASHI, Sakurako**
  **Settsu-shi, Osaka 566-8510 (JP)**
• **NISHI, Mitsuharu**
  **Settsu-shi, Osaka 566-8510 (JP)**
• **YOSHIDA, Hiroshi**
  **Settsu-shi, Osaka 566-8510 (JP)**
• **SANO, Yoshihiko**
  **Settsu-shi, Osaka 566-8510 (JP)**

(74) Representative: **Prüfer & Partner mbB**
**Patentanwälte · Rechtsanwälte**
**Sohnckestraße 12**
**81479 München (DE)**

(54) **INFLAMMATORY CYTOKINE PRODUCTION INHIBITION DEVICE**

(57)     A proinflammatory cytokine production inhibitory apparatus for inhibiting production of a proinflammatory cytokine by magnetic stimulation of cells, the proinflammatory cytokine production inhibitory apparatus comprising:
an electromagnetic wave production member to produce an electromagnetic wave at a proinflammatory cytokine production inhibitory frequency so as to expose the cells to an alternating magnetic field at the proinflammatory cytokine production inhibitory frequency;
a frequency control member to control the proinflammatory cytokine production inhibitory frequency of the electromagnetic wave to 100 to 450 MHz so as to inhibit production of the proinflammatory cytokine by magnetic stimulation with the alternating magnetic field at the proinflammatory cytokine production inhibitory frequency; and
an irradiation member to irradiate the cells with the alternating magnetic field at the proinflammatory cytokine production inhibitory frequency.

FIG.1

FREQUENCY CHARACTERISTICS OF INFLAMMATION INHIBITORY EFFECT

DECREMENT RATE OF PROINFLAMMATORY CYTOKINES (%)

FREQUENCY (MHz)

**Description**

TECHNICAL FIELD

The present invention relates to a proinflammatory cytokine production inhibitory apparatus for inhibiting production of a proinflammatory cytokine by magnetic stimulation of cells.

BACKGROUND ART

Conventionally, treatment of inflammation is performed mainly by drug therapy with anti-inflammatory drugs, and as the anti-inflammatory drugs, steroidal anti-inflammatory drugs and nonsteroidal anti-inflammatory drugs are widely used. However, steroidal anti-inflammatory drugs cause many adverse effects such as immunosuppression, adrenal gland atrophy, gastrointestinal disorders, cataract, glaucoma, and moonface, where adrenal gland atrophy can be caused by a long-time, high-dose administration, and non-taper discontinuation of steroid can cause adrenal insufficiency. In addition, early stages of steroidal anti-inflammatory drug administration can be often accompanied by frustration feelings, insomnia, indigestion, diarrhea, nausea, increased appetite, and the like; and adverse effects caused by long-term administration of steroidal anti-inflammatory drugs include a unique reaction called abnormal fat deposition, and those caused by high-dose administration and/or long-term administration include adrenal insufficiency, development of various infectious diseases such as viral hepatitis, increased blood glucose, osteopsathyrosis, gastric ulcer, depression, ocular hypertension, arteriosclerosis, and thrombosis. On the other hand, nonsteroidal anti-inflammatory drugs have common adverse effects including gastrointestinal disorders, hepatic disorders, renal disorders, hematopoietic disorders, SJ (Stevens-Johnson) syndrome, and aspirin-induced asthma. Due to all these reasons, development of a means for inflammation treatment without adverse effects has been demanded.

Meanwhile, there is a conventional technique that involves irradiating a living thing with a magnetic field to stimulate cells and nerves of the living thing. For example, Mitsuharu Nishi, et al. ("Development of therapy for neuropathic pain by stimulation with alternating magnetic field" (Abstract) (NPL 1) recite that they used a magnetic stimulation apparatus having a carrier wave at 50 MHz to 300 MHz and a mixed frequency of modulation of 1 k to 3 kHz and 6 to 10 Hz to investigate the influence on thermal hyperalgesia and spinal cord cell disorders. NPL 1 also recites that magnetic stimulation of injured peripheral nerves induced exocytosis from peripheral sensory nerves and sympathetic nerves and caused a release of nerve growth factors, suggesting potential involvement in restoration of the function of the sensory nervous system.

Also, U.S. Patent No. 5723001 (PTL 1) discloses an apparatus for therapeutically treating human body tissue with pulsed radio frequency electromagnetic radiation. The apparatus disclosed by PTL 1 generates pulse bursts at frequencies between 1 to 100 MHz with 100 to 100,000 pulses per burst, where the repetition rate of such pulse bursts varies from 0.01 to 1000 Hz (lines 49-60 in the third section of PTL 1) and the pulse envelope is regular, irregular, or random (lines 33-37 in the third section of PTL 1).

However, no technique is previously known that makes reference to the correlation between magnetic stimulation with an alternating magnetic field of equal to or more than 100 MHz and alleviation of inflammation.

CITATION LIST

PATENT LITERATURE

PTL 1: U.S. Patent No. 5723001

NON PATENT LITERATURE

NPL 1: Mitsuharu Nishi, et al. "Development of therapy for neuropathic pain by stimulation with alternating magnetic field" (Abstract)

SUMMARY OF INVENTION

TECHNICAL PROBLEM

The present invention aims at providing a novel proinflammatory cytokine production inhibitory apparatus that utilizes magnetic stimulation with an alternating magnetic field of equal to or more than 100 MHz.

SOLUTION TO PROBLEM

The present invention relates to
a proinflammatory cytokine production inhibitory apparatus for inhibiting production of a proinflammatory cytokine by magnetic stimulation of cells, the proinflammatory cytokine production inhibitory apparatus comprising:

an electromagnetic wave production member to produce an electromagnetic wave at a proinflammatory cytokine production inhibitory frequency so as to expose the cells to an alternating magnetic field at the proinflammatory cytokine production inhibitory frequency;
a frequency control member to control the proinflammatory cytokine production inhibitory frequency of the electromagnetic wave to 100 to 450 MHz so as to inhibit production of the proinflammatory cytokine by magnetic stimulation with the alternating magnetic field at the proinflammatory cytokine production inhibitory frequency; and
an irradiation member to irradiate the cells with the alternating magnetic field at the proinflammatory cytokine production inhibitory frequency.

In the proinflammatory cytokine production inhibitory apparatus according to the present invention, the proinflammatory cytokine is preferably at least one selected from the group consisting of tumor necrosis factor (TNF), interleukin 6 (IL-6), and interleukin 1β (IL-1β).
In the proinflammatory cytokine production inhibitory apparatus according to the present invention, it is preferable that the proinflammatory cytokine be TNF and the proinflammatory cytokine production inhibitory frequency be within the range of 100 to 450 MHz, more preferably within the range of 150 to 300 MHz or within the range of 400 to 450 MHz.
In the proinflammatory cytokine production inhibitory apparatus according to the present invention, it is preferable that the proinflammatory cytokine be IL-6 and the proinflammatory cytokine production inhibitory high frequency be within the range of 150 to 450 MHz, more preferably within the range of 150 to 250 MHz or within the range of 350 to 450 MHz.
In the proinflammatory cytokine production inhibitory apparatus according to the present invention, it is preferable that the proinflammatory cytokine be 1L-1β and the proinflammatory cytokine production inhibitory frequency be within the range of 150 to 450 MHz, more preferably within the range of 150 to 250 MHz.

ADVANTAGEOUS EFFECTS OF INVENTION

According to the present invention, a human body (an affected area) is irradiated with an alternating magnetic field at a proinflammatory cytokine production inhibitory frequency, and thereby the alternating magnetic field permeates into the body and reaches cells to reduce proinflammatory cytokine production. As a result, inflammation is reduced, pathological conditions are alleviated, and the pain caused by the inflammation is reduced, where adverse effects that can be caused by administration of conventional anti-inflammatory drugs are avoided.

BRIEF DESCRIPTION OF DRAWINGS

Fig. 1 is a graph showing results of Experiment Example 1, where the vertical axis represents the decrement rate (%) of proinflammatory cytokines, and the horizontal axis represents frequency (MHz).
Fig. 2 is a graph showing results of TNF.
Fig. 3 is a schematic view illustrating a specific example of a proinflammatory cytokine production inhibitory apparatus according to the present invention.
Fig. 4 is a schematic view illustrating an irradiation member of a proinflammatory cytokine production inhibitory apparatus according to an embodiment of the present invention, where (a) is a top view, (b) is a rear view, and (c) is an A-A cross-sectional view.

DESCRIPTION OF EMBODIMENTS

A proinflammatory cytokine production inhibitory apparatus according to the present invention is an apparatus for inhibiting production of proinflammatory cytokines by magnetic stimulation of cells. As demonstrated by Experiment Examples described below, the inventors of the present invention have found that it is possible to markedly inhibit proinflammatory cytokine production in cells by magnetic stimulation with an alternating magnetic field of equal to or more than 100 MHz.

The cells according to the present invention are a type of cells that have the ability to produce proinflammatory cytokines (immunocompetent cells), and examples thereof include dendritic cells, macrophages, T cells (helper T cells, killer T cells, regulatory T cells), B cells, natural killer (NK) cells, neutrophils, eosinophils, basophils, and neural cells (such as neurons and glial cells, for example).

In the present invention, the proinflammatory cytokines are not particularly limited as long as each of them is widely known as a proinflammatory cytokine, and examples thereof include TNF, IL-6, interleukin 1α (IL-1α), IL-1β, interleukin 8 (IL-8), interleukin 11 (IL-11), interleukin 17 (IL-17), interleukin 18 (IL-18), interferon-α (IFN-α), interferon-β (IFN-β), and interferon-γ (IFN-γ); among these, at least one selected from the group consisting of TNF, IL-6, and IL-1β is preferable because these are key proinflammatory cytokines that are increased in inflammatory diseases and often used as inflammation markers.

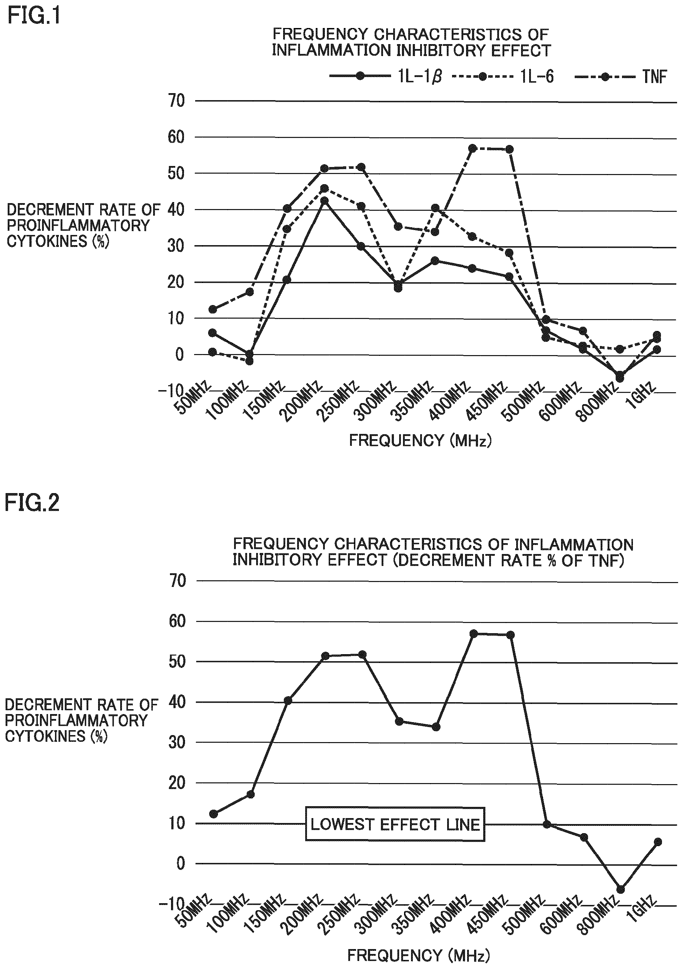

Here, Fig. 1 is a graph showing results of Experiment Example 1, which is described below. In Fig. 1, the vertical axis represents the decrement rate (%) of proinflammatory cytokines (TNF, IL-6, IL-1β, and the horizontal axis represents frequency (MHz) (50 MHz, 100 MHz, 150 MHz, 200 MHz, 250 MHz, 300 MHz, 350 MHz, 400 MHz, 450 MHz, 500 MHz, 600 MHz, 800 MHz, and 1 GHz). Fig. 1 indicates that within the range of frequency of 100 to 450 MHz ("the proinflammatory cytokine production inhibitory frequency" according to the present invention), proinflammatory cytokines TNF, IL-6, and IL-1β markedly decreased.

Referring to the results shown in Fig. 1, in the proinflammatory cytokine production inhibitory apparatus according to the present invention, when the proinflammatory cytokine is TNF, the proinflammatory cytokine production inhibitory frequency is preferably within the range of 100 to 450 MHz, more preferably within the range of 150 to 300 MHz or within the range of 400 to 450 MHz.

Also referring to the results shown in Fig. 1, in the proinflammatory cytokine production inhibitory apparatus according to the present invention, when the proinflammatory cytokine is IL-6, the proinflammatory cytokine production inhibitory frequency is preferably within the range of 150 to 450 MHz, more preferably within the range of 150 to 250 MHz or within the range of 350 to 450 MHz.

Also referring to the results shown in Fig. 1, in the proinflammatory cytokine production inhibitory apparatus according to the present invention, it is preferable that the proinflammatory cytokine be 1L-1β and the proinflammatory cytokine production inhibitory frequency be within the range of 150 to 450 MHz, more preferably within the range of 150 to 250 MHz.

Here, Fig. 2 is a graph showing results of TNF, where the vertical axis represents the decrement rate (%) of the proinflammatory cytokine (TNF), and the horizontal axis represents frequency (MHz) (50 MHz, 100 MHz, 150 MHz, 200 MHz, 250 MHz, 300 MHz, 350 MHz, 400 MHz, 450 MHz, 500 MHz, 600 MHz, 800 MHz, and 1 GHz). The graph shows TNF which exhibited the highest inflammation inhibitory rate among the three types of proinflammatory cytokines shown in Fig. 1, together with a lowest effect line (the TNF data used here is the same as the TNF data used in Fig. 1). Here, Alissar Nehme and Jeffrey Edelman, "Dexamethasone Inhibits High Glucose-, TNF-α-, and IL-1β-Induced Secretion of Inflammatory and Angiogenic Mediators from Retinal Microvascular Pericytes", Investigative Ophthalmology & Visual Science, (2008), vol. 49, No. 5 give a TNF concentration-effect curve in Graph A of Figure 6, on which dexamethasone (a conventionally-known anti-inflammatory drug) shows an inhibitory rate of 10% at a concentration of $1.0 \times 10^{-7}$ M (=39.2 ng/mL). Meanwhile, the package insert of over-the-counter dexamethasone "Decadron Tablets 4 mg" (Nichi-Iko Pharmaceutical Co., Ltd.) gives, in Section "16. Pharmacokinetics", a graph of plasma dexamethasone concentrations for oral administration of Decadron Tablets 4 mg, on which a plasma concentration of 39.2 ng/mL is the maximum plasma concentration at about 1 hour after oral administration of Decadron Tablets 4 mg. From these information, it is indicated that 10% inhibition of TNF is sufficient as the effect of an anti-inflammatory drug. The above-mentioned package insert recites in Section "6. Dosage regimen and dose" that "<Other notes common to all indications> To an adult, under ordinary circumstances, give 0.5 to 8 mg (as dexamethasone) per day orally in 1 to 4 divided doses."; so, with the assumption that the proinflammatory cytokine production inhibitory apparatus according to the present invention exhibits 10% inhibition (90% remaining) at a single stimulation, two stimulations per day by the proinflammatory cytokine inhibitory apparatus according to the present invention can achieve nearly 20% inhibition calculated based on 10% inhibition per single stimulation and 90%×90%=81% remaining. Furthermore, three stimulations per day by the proinflammatory cytokine inhibitory apparatus according to the present invention can achieve nearly 30% inhibition, which is more effective than the anti-inflammatory drug. Hence, the proinflammatory cytokine inhibitory apparatus according to the present invention is expected to exhibit a sufficient level of proinflammatory cytokine inhibitory effect also in clinical settings with 10% inhibition per single stimulation, which is shown by the lowest effect line in Fig. 2.

Referring to Fig. 2, it is indicated as follows: marked TNF reducing effects were exhibited at 100 MHz or higher; and, at between 250 to 350 MHz, although not as high as the highest effects exhibited at 200 MHz, 400 MHz, and 450 MHz, a sufficient level of effect as compared to conventionally known anti-inflammatory drugs was exhibited considering the above-mentioned lowest effect line.

According to the present invention, a human body (an affected area) is irradiated with an alternating magnetic field at a proinflammatory cytokine production inhibitory frequency, and thereby the alternating magnetic field permeates into the body and reaches cells to reduce proinflammatory cytokine production in the cells. As a result, inflammation is reduced, pathological conditions are alleviated, and the pain caused by the inflammation is reduced, where adverse effects that can be caused by administration of conventional anti-inflammatory drugs are avoided.

The proinflammatory cytokine production inhibitory apparatus according to the present invention comprises: an electromagnetic wave production member to produce an electromagnetic wave at a proinflammatory cytokine production inhibitory frequency so as to expose the cells to an alternating magnetic field at the proinflammatory cytokine production inhibitory frequency; a frequency control member to control the proinflammatory cytokine production inhibitory frequency of the electromagnetic wave so as to inhibit production of the proinflammatory cytokine by magnetic stimulation with the alternating magnetic field at the proinflammatory cytokine production inhibitory frequency; and an irradiation member to irradiate the cells with the alternating magnetic field at the proinflammatory cytokine production inhibitory frequency.

The electromagnetic wave production member comprises a signal production member, a signal amplification member, and a signal wave output member equipped with an electromagnetic wave production antenna, for example. The signal production member produces a signal of equal to or more than 100 MHz. The signal produced at the signal production member is supplied to the signal amplification member, amplified at the signal amplification member, and output to the electromagnetic wave production antenna. The electromagnetic wave production antenna may be, for example, a coil formed on a print board by circuit printing, and it produces an electromagnetic wave corresponding to the signal from the signal amplification member. The electromagnetic wave includes an alternating magnetic field and an alternating electric field.

The frequency control member is configured to control the above-mentioned operation at the signal wave output member to control the proinflammatory cytokine production inhibitory frequency of the electromagnetic wave. The frequency control member, where its circuit is configured with the use of a graphics processing unit (GPU) and/or the like, for example, is displayed on a liquid crystal display (LCD) and/or the like of a touch panel display of an image display member, and can be controlled by a user of the proinflammatory cytokine production inhibitory apparatus through operation of the image display member. For example, a position on a touch panel of the touch panel display touched by a finger of a user of the proinflammatory cytokine production inhibitory apparatus can be detected based on the electrostatic changes at the position, and in response to the touched position, according to the signal indicative of a command input via an operation button displayed on the LCD and/or the like, control can be executed according to the command from the user, on operations such as production and output of signal waves at the signal wave output member as well as on a high-frequency-wave alternating magnetic field which is to be produced at the irradiation member.

The irradiation member is configured to irradiate the cells with the alternating magnetic field at the proinflammatory cytokine production inhibitory frequency produced from the above-mentioned electromagnetic wave production antenna. Preferably, the irradiation member is, for example, a flexible, plate-shaped member having the electromagnetic wave production antenna inside itself, and is manually deformable along the contour of the target site to which a user of the proinflammatory cytokine inhibitory apparatus intends to direct the irradiation.

In the following, a specific example of the proinflammatory cytokine production inhibitory apparatus according to the present invention will be described referring to drawings. It should be noted that the drawings are schematic illustration and may be different from reality. Moreover, the below explanation is given for the purpose of specifically describing the technical principles of the present invention and not intended to restrict the configuration to those described below, and the technical principles of the present invention may be modified in various ways within the technical scope described in claims.

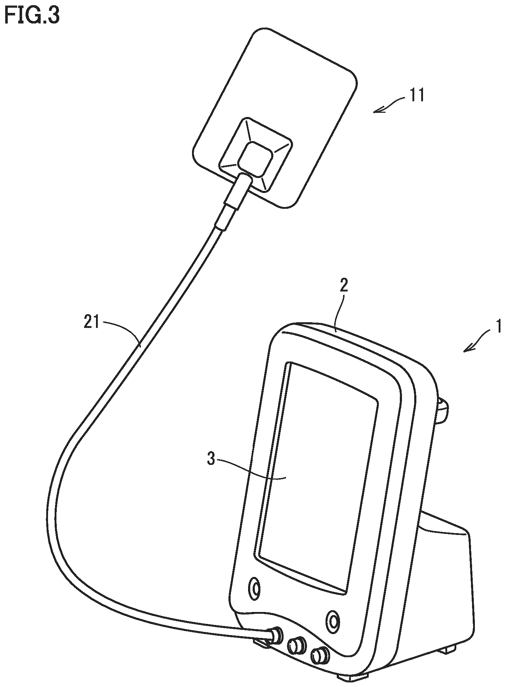

Here, Fig. 3 is a schematic view illustrating a specific example of a proinflammatory cytokine production inhibitory apparatus according to the present invention. The proinflammatory cytokine production inhibitory apparatus illustrated in Fig. 3 comprises an apparatus main body 1 and a probe 11 connected to apparatus main body 1 via a signal cable 21 (corresponding to the above-mentioned irradiation member), and, in addition, a power cable (not shown) is detachably inserted in apparatus main body 1.

Apparatus main body 1 of the proinflammatory cytokine production inhibitory apparatus illustrated in Fig. 3 has a casing 2 made of resin, a touch panel display 3 housed inside the front portion of casing 2 in a manner to face upward in a slanting position and exposed from an opening at the front side of casing 2 (corresponding to the above-mentioned touch panel display of the frequency control member), a signal wave output member housed inside the upper back portion of casing 2, and a power supply member housed inside the lower back portion of casing 2. An alarm stop button and a power switch button are provided on the front side of casing 2 of apparatus main body 1, next to each other below the opening. Below these buttons, a plurality of sockets for plugs of signal cables 21 are provided next to each other allowing for connection of a plurality of probes 11 to apparatus main body 1.

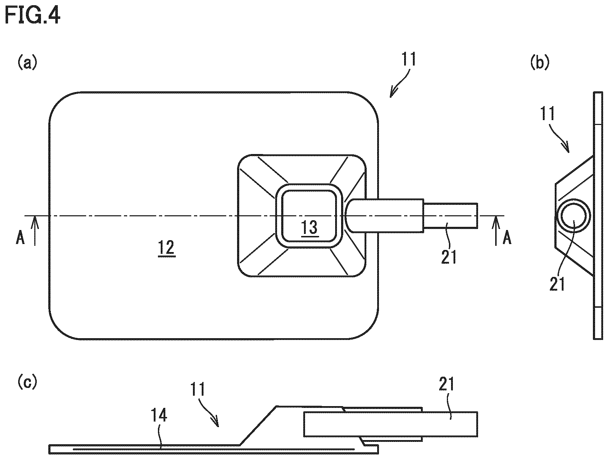

Fig. 4 gives a top view (a), a rear view (b), and an A-A cross-sectional view (c) of probe 11 of the proinflammatory cytokine production inhibitory apparatus according to the present embodiment. In this embodiment, probe 11 comprises a housing 12 having a substantially four-sided shape and made of flexible material, as well as a circuit member 13 having a truncated pyramid shape for protecting an electrical circuit and the like. Signal cable 21 juts out from the back of the circuit member.

As illustrated in Fig. 4(c), probe 11 has a flexible thin plate 14 which is a print board having a coil and/or an electrical circuit on itself, housed inside the housing 12 which is made of soft resin such as elastomer or rubber material. Probe 11 can be produced by resin molding such as injection molding and/or RIM molding. During resin molding, self-heating occurs, so for the purpose of protecting the electrical circuit and the like, it is preferable to apply resin molding to circuit member 13 in advance.

On the upper side, for example, of the print board, a high-frequency wave output coil (corresponding to the above-mentioned electromagnetic wave production antenna) is provided in the form of a ring-shaped disk, and outside of it, a high-frequency wave detection coil is provided in the form of a circle. Another high-frequency wave output coil is provided on the lower side of the print board, at a position symmetric with respect to a plane to the high-frequency wave output coil provided on the upper side, so as to make high-frequency wave currents flow in the same direction on both the upper side and the lower side of the print board. It would be obvious that both the high-frequency wave output coil and the high-frequency wave detection coil may have a four-sided shape.

Probe 11 is not particularly limited in terms of its shape, and when it is a flat plate having a substantially four-sided shape, or a substantially rectangular shape in particular, as in the example illustrated in Fig. 4, it is easy to put it on the target and easy to secure it with tape, which is advantageous.

Preferably, flexible thin plate 14 is a film-shaped print board, and it has a base layer made of a bendable film formed of electrically insulating material and also has an adhesive layer as needed. For the base layer, polyimide resin, polyester resin, polyamide paper base epoxy resin, glass fabric base epoxy resin, glass base BT resin, and/or the like can be used. Each coil may be a conductor that is printed or etched on print board 14 which is a metal foil sheet and/or the like.

In the proinflammatory cytokine production inhibitory apparatus according to the present invention, the power supply member has a circuit (not shown) configured with CPU and two AC-DC converters, for example; it charges a battery, by obtaining certain DC powers via the above-mentioned two AC-DC converters from 100-V AC utility power supplied through a power cable (not shown) and then connecting these DC powers in series to obtain charging voltage for the battery; and it also supplies certain DC power, which is obtained by lowering the voltage of the above-mentioned DC powers through a switching power supply and a linear regulator, to the signal wave output member and an image control member of apparatus main body 1, as well as to an operation-state detection member of probe 11, each as DC power at a voltage necessary for each of them. During normal use of the proinflammatory cytokine production inhibitory apparatus when it is not connected to the power cable, the power supply member supplies DC power from the battery to the signal wave output member and the image control member of apparatus main body 1 as well as to the operation-state detection member of probe 11, after the voltage is lowered by the switching power supply and the linear regulator to the voltage necessary for each of them, enabling portable use of the proinflammatory cytokine production inhibitory apparatus.

In the following, a more detailed description will be given of the present invention by way of Experiment Examples, but these are not intended to limit the scope of the present invention.

<Experiment Example 1>

(Cell Incubation)

MG5 cells (IFO50520) Lot: 11132008, which were a cell line derived from mouse microglia, and mouse astrocyte-like cell line A1 cells (IFO50519) Lot: 02232011, which were to be used for preparation of culture supernatant necessary for incubation of the MG5 cells, were obtained from JCRB Cell Bank. The MG5 cells were incubated in a medium which was a 7:3 mixture of culture supernatant of the A1 cells and DMEM containing 10% fetal bovine serum. The A1 cells were incubated in DMEM containing 10% fetal bovine serum.

(Inflammatory Stimulation and Magnetic Stimulation)

After 80% confluency was confirmed, the MG5 cells were divided into three groups specified below.

·Control (non-stimulation) group
·LPS-stimulation group
·LPS-stimulation magnetic-stimulation group

The LPS (lipopolysaccharide) used here was a product from Sigma, which was used as inflammatory stimulation at a concentration of 0.1 ng/ml and incubated for 3 hours (LPS stimulation).

For the magnetic stimulation, a function generator 81160A (manufactured by Keysight Technologies (consolidated company)) was used, and magnetic irradiation was given to the LPS-stimulation magnetic-stimulation group starting at one hour prior to LPS stimulation, so, as a result, magnetic irradiation was given for consecutive four hours in total. As the magnetic stimulation, high-frequency waves of 50 MHz, 100 MHz, 150 MHz, 200 MHz, 250 MHz, 300 MHz, 350 MHz, 400 MHz, and 450 MHz were used. The waveform of the high-frequency waves was a sine wave, and the output was v(max)=5.0 Vpp for 50 MHz, 100 MHz, 150 MHz, 200 MHz, 250 MHz, and 300 MHz and v(max)=3.0 Vpp for 350 MHz, 400 MHz, and 450 MHz.

After the completion of stimulation, total RNA of the cells was extracted with the use of Pure Link RNA Mini Kit (manufactured by Thermo Fisher SCIENTIFIC). By RT-qPCR, expression of TNF mRNA (Mm00443258_m1), IL-6 mRNA (Mm00446190_m1), and IL-1β mRNA (Mm00434228_m1) was investigated.

(Analysis of Expression Amount of mRNA of Each Cytokine by Reverse Transcription-qPCR)

RNA extraction was carried out according to the protocol of the product, and after RNA elution, the RNA concentration was measured with a microspectrophotometer (manufactured by Implen).

(Genomic DNA Removal Reaction and Reverse Transcription Reaction)

Genomic DNA removal and reverse transcription were carried out with the use of PrimeScript RT reagent Kit with gDNA Eraser (Perfect Real Time) (manufactured by Takara Bio), according to the protocol of the product.

After reverse transcription, the reaction solution was diluted to a concentration of 2.5 ng/$\mu$l, and 2 $\mu$l (5 ng) was used as a template for PCR.

(qPCR Reaction)

qPCR was carried out with the use of Taqman Assays (manufactured by Thermo Fisher SCIENTIFIC), where real-time PCR reaction solution was prepared for each of GAPDH (Mm99999915_g1) and proinflammatory cytokines (TNF, IL-6, and IL-1β), and a thermal cycler was programed as specified below to carry out the reaction.

[Table 1]

| 50°C | 2 minutes | (Activation by UNG incubation) | |
|------|-----------|-------------------------------|---|
| 95°C | 20 seconds | Polymerase activation | |
| 95°C | 1 second | PCR·(Heat-denaturation) | 40 cycles |
| 60°C | 20 seconds | PCR·(Annealing extention) | |

From the Ct value, a relative value relative to GAPDH was calculated.

(Calculation of Relative Rate)

·ΔCt(control)=(Ct control)-(Ct control GAPDH)

·ΔCt(test sample)=(Ct test)-(Ct test GAPDH)

·ΔΔCt=ΔCt(test sample)-ΔCt(control)

·Relative rate=$2^{-\Delta\Delta Ct}$

Results of the LPS-stimulation magnetic-stimulation group for TNF, IL-6, and IL-Iβ are given in Fig. 1, plotting inflammation inhibitory rates at respective frequencies. In Fig. 1, the vertical axis represents the decrement rate (%) of the proinflammatory cytokines (TNF, IL-6, IL-1β), and the horizontal axis represents frequency (MHz) (50 MHz, 100 MHz, 150 MHz, 200 MHz, 250 MHz, 300 MHz, 350 MHz, 400 MHz, 450 MHz, 500 MHz, 600 MHz, 800 MHz, and 1 GHz). Referring to Fig. 1, it is indicated that TNF, IL-6, and IL-1β markedly decreased at frequencies within the range of 100 to 450 MHz.

Referring to the results shown in Fig. 1, TNF decreased at 100 to 450 MHz, particularly markedly decreased at 150 to 300 MHz and at 400 to 450 MHz. IL-6 decreased at 150 to 450 MHz, particularly markedly decreased at 150 to 250 MHz and 350 to 450 MHz. 1L-1β decreased at 150 to 450 MHz, particularly markedly decreased at 150 to 250 MHz.

Fig. 2 is a graph showing results of TNF, where the vertical axis represents the decrement rate (%) of the proinflammatory cytokine (TNF), and the horizontal axis represents frequency (MHz) (50 MHz, 100 MHz, 150 MHz, 200 MHz, 250 MHz, 300 MHz, 350 MHz, 400 MHz, 450 MHz, 500 MHz, 600 MHz, 800 MHz, and 1 GHz). Referring to Fig. 2, it is indicated that marked TNF reducing effects were exhibited at 100 MHz or higher, and, as mentioned above, at between 250 to 350 MHz, although not as high as the highest effects exhibited at 200 MHz, 400 MHz, and 450 MHz, a sufficient level of effect as compared to conventionally known anti-inflammatory drugs was exhibited considering the lowest effect line.

It should be construed that the embodiments disclosed herein are given by way of illustration in all respects, not by way of limitation. It should also be construed that the scope of the present invention is interpreted by the terms of the appended claims, not by the above description, and encompasses all modifications and variations equivalent in meaning and scope to the claims.

REFERENCE SIGNS LIST

1 main body of proinflammatory cytokine production inhibitory apparatus, 2 casing, 3 display (touch panel display), 11 probe, 12 housing, 13 circuit member, 14 flexible thin plate (print board), 21 signal cable.

**Claims**

1. A proinflammatory cytokine production inhibitory apparatus for inhibiting production of a proinflammatory cytokine by magnetic stimulation of cells, the proinflammatory cytokine production inhibitory apparatus comprising:

   an electromagnetic wave production member to produce an electromagnetic wave at a proinflammatory cytokine production inhibitory frequency so as to expose the cells to an alternating magnetic field at the proinflammatory cytokine production inhibitory frequency;
   a frequency control member to control the proinflammatory cytokine production inhibitory frequency of the electromagnetic wave to 100 to 450 MHz so as to inhibit production of the proinflammatory cytokine by magnetic stimulation with the alternating magnetic field at the proinflammatory cytokine production inhibitory frequency; and
   an irradiation member to irradiate the cells with the alternating magnetic field at the proinflammatory cytokine production inhibitory frequency.

2. The proinflammatory cytokine production inhibitory apparatus according to claim 1, wherein the proinflammatory cytokine is at least one selected from the group consisting of tumor necrosis factor, interleukin 6, and interleukin 1β.

3. The proinflammatory cytokine production inhibitory apparatus according to claim 1 or 2, wherein the proinflammatory cytokine is a tumor necrosis factor, and the proinflammatory cytokine production inhibitory frequency is within the range of 100 to 450 MHz.

4. The proinflammatory cytokine production inhibitory apparatus according to claim 3, wherein the proinflammatory cytokine production inhibitory frequency is within the range of 150 to 300 MHz or within the range of 400 to 450 MHz.

5. The proinflammatory cytokine production inhibitory apparatus according to claim 1 or 2, wherein the proinflammatory cytokine is interleukin 6, and the proinflammatory cytokine production inhibitory frequency is within the range of 150 to 450 MHz.

6. The proinflammatory cytokine production inhibitory apparatus according to claim 5, wherein the proinflammatory cytokine production inhibitory frequency is within the range of 150 to 250 MHz or within the range of 350 to 450 MHz.

7. The proinflammatory cytokine production inhibitory apparatus according to claim 1 or 2, wherein the proinflammatory cytokine is interleukin 1β, and the proinflammatory cytokine production inhibitory frequency is within the range of 150 to 450 MHz.

8. The proinflammatory cytokine production inhibitory apparatus according to claim 7, wherein the proinflammatory cytokine production inhibitory frequency is within the range of 150 to 250 MHz.

FIG.1

FREQUENCY CHARACTERISTICS OF
INFLAMMATION INHIBITORY EFFECT

●——— 1L-1β ----●---- 1L-6 ——●— TNF

DECREMENT RATE OF
PROINFLAMMATORY
CYTOKINES (%)

FREQUENCY (MHz)

FIG.2

FREQUENCY CHARACTERISTICS OF INFLAMMATION
INHIBITORY EFFECT (DECREMENT RATE % OF TNF)

DECREMENT RATE OF
PROINFLAMMATORY
CYTOKINES (%)

LOWEST EFFECT LINE

FREQUENCY (MHz)

FIG.3

FIG.4

(a)

11

A

12

13

21

A

(b)

11

21

(c)

11

14

21

# EP 4 509 168 A1

<div align="center">

**INTERNATIONAL SEARCH REPORT**

</div>

| | International application No. |
|---|---|
| | **PCT/JP2023/014178** |

---

**A.    CLASSIFICATION OF SUBJECT MATTER**

*A61N 2/04*(2006.01)i
FI:    A61N2/04

According to International Patent Classification (IPC) or to both national classification and IPC

---

**B.    FIELDS SEARCHED**

Minimum documentation searched (classification system followed by classification symbols)
A61N2/04

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

---

**C.    DOCUMENTS CONSIDERED TO BE RELEVANT**

| Category* | Citation of document, with indication, where appropriate, of the relevant passages | Relevant to claim No. |
|---|---|---|
| X | WO 2008/056414 A1 (MEDICAL APPLIANCE CO., LTD.) 15 May 2008 (2008-05-15) paragraphs [0045]-[0047], [0077]-[0086], [0130], [0131], [0151]-[0157], [0216], [0222]-[0229] | 1-8 |
| X | US 2011/0034855 A1 (BOARD OF REGENTS, THE UNIVERSITY OF TEXAS SYSTEM) 10 February 2011 (2011-02-10) paragraphs [0003]-[0011], [0054] | 1-8 |
| X | US 2010/0292564 A1 (CANTILLON MURPHY, Padraig J.) 18 November 2010 (2010-11-18) paragraphs [0038], [0059] | 1-8 |

---

☐ Further documents are listed in the continuation of Box C.        ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **29 May 2023** | **13 June 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

12

**INTERNATIONAL SEARCH REPORT**
Information on patent family members

International application No.

**PCT/JP2023/014178**

| Patent document cited in search report | | | Publication date (day/month/year) | Patent family member(s) | Publication date (day/month/year) |
|---|---|---|---|---|---|
| WO | 2008/056414 | A1 | 15 May 2008 | US 2009/0326315 A1 paragraphs [0070]-[0072], [0102]-[0111], [0155], [0156], [0174]-[0180], [0237], [0242]-[0250] EP 2081645 B1 ES 2407413 T3 CN 101534903 A HK 1185577 A1 | |
| US | 2011/0034855 | A1 | 10 February 2011 | (Family: none) | |
| US | 2010/0292564 | A1 | 18 November 2010 | (Family: none) | |

Form PCT/ISA/210 (patent family annex) (January 2015)

**EP 4 509 168 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5723001 A

**Non-patent literature cited in the description**

- **MITSUHARU NISHI et al.** *Development of therapy for neuropathic pain by stimulation with alternating magnetic field*

- **ALISSAR NEHME** ; **JEFFREY EDELMAN**. Dexamethasone Inhibits High Glucose-, TNF-α-, and IL-1β-Induced Secretion of Inflammatory and Angiogenic Mediators from Retinal Microvascular Pericytes. *Investigative Ophthalmology & Visual Science*, 2008, vol. 49 (5)